(19) Europäisches Patentamt
European Patent Office
Office européen des brevets

(11) EP 0 599 114 B1

(12) EUROPEAN PATENT SPECIFICATION

(45) Date of publication and mention of the grant of the patent:
**15.01.1997 Bulletin 1997/03**

(51) Int. Cl.$^6$: **C07C 401/00**, C07J 51/00, A61K 31/59, C07F 7/18

(21) Application number: **93117951.9**

(22) Date of filing: **05.11.1993**

# (54) Vitamin D3 analogs

Vitamin D3 Derivate

Analogues de la vitamine D3

(84) Designated Contracting States:
**AT BE CH DE DK ES FR GB GR IE IT LI LU MC NL PT SE**

(30) Priority: **20.11.1992 US 979133**

(43) Date of publication of application:
**01.06.1994 Bulletin 1994/22**

(73) Proprietor: **F. HOFFMANN-LA ROCHE AG**
**4002 Basel (CH)**

(72) Inventors:
- **Doran, Thomas I.**
  **West Orange, N.J. 07052 (US)**
- **McLane, John Arthur**
  **New Haven, CT 06516 (US)**
- **Okabe, Masami**
  **Nutley, N.J. 07110 (US)**
- **Scalone, Michelangelo**
  **CH-4127 Birsfelden (CH)**
- **Uskokovic, Milan Radoje**
  **Upper Montclair, N.J. 07043 (US)**

(74) Representative: **Mahé, Jean et al**
**F.Hoffmann-La Roche AG**
**Patent Department (PLP),**
**124 Grenzacherstrasse**
**4070 Basel (CH)**

(56) References cited:
**EP-A- 0 321 572**
**EP-A- 0 325 279**
**EP-A- 0 377 743**
**EP-A- 0 398 217**
**WO-A-92/14746**

- **CANCER RESEARCH vol. 50, no. 21 , 1 November 1990 , WASHINGTON, US pages 6857 - 6864 A. W. NORMAN ET AL 'Structure-function studies on analogues of 1-alpha-25-dihydroxyvitamin D3: differential effects on leukemic cell growth, differentiation, and intestinal calcium absorption.'**
- **THE JOURNAL OF NUTRITIONAL BIOCHEMISTRY vol. 4, no. 1 , January 1993 , BOSTON, US pages 49 - 57 T. C. CHEN ET AL 'An evaluation of 1,25-dihydroxyvitamin D3 analogues on the proliferation and differentiation of cultured human keratinocytes, calcium metabolism and the differentiation of human HL-60 cells.'**

EP 0 599 114 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.13/3.4

## Description

The invention relates to compounds of the formula

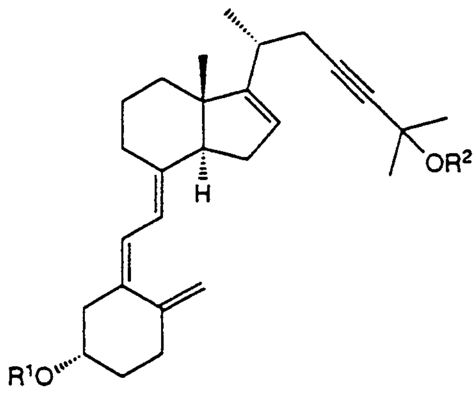

I

wherein $R^1$ and $R^2$ are hydrogen or acyl; provided that only one of $R^1$ or $R^2$ is hydrogen. The corresponding diols, wherein $R^1$ and $R^2$ would stand for hydrogen are described in EP-A-398 217, EP-A-325 279 and Cancer Res. 50 (1990) 6857-64.

The invention further relates to the above compounds for use as therapeutically active agents and to the use of said compounds for the manufacture of medicaments for the treatment of hyperproliferative skin diseases, such as psoriasis, and for the treatment of sebaceous gland diseases, such as acne and seborrheic dermatitis. The invention also relates to a composition comprising an effective amount of a compound of formula I, and further to a process for preparing compounds of formula I, and to intermediates of formulae IV, VI, VII, VIII and XIII, as defined below.

As used herein, the term "acyl" denotes a group of the formula $R^3CO$- wherein $R^3$ is lower alkyl or aryl, which may be unsubstituted or substituted by one or more substituents selected from the group consisting of lower alkyl, aryl, alkoxy and acyloxy. The term "lower alkyl" denotes a straight or branched-chain alkyl group containing 1 to 4 carbon atoms, e.g., methyl, ethyl, propyl, isopropyl and butyl. The term "aryl" denotes a group derived from an aromatic hydrocarbon. The above compounds stimulate differentiation and decrease proliferation of human keratinocytes. Accordingly, they are useful as agents in the treatment of hyperproliferative skin disorders such as psoriasis, basal cell carcinomas, disorders of keratinization, and keratosis. They are also useful as agents for the treatment of sebaceous gland diseases such as acne or seborrheic dermatitis.

Preferably in the compounds of formula I, $R^1$ is acyl, preferably $R^3CO$ wherein $R^3$ is lower alkyl or aryl and $R^2$ is hydrogen or acetyl. Preferred compounds of formula I of the invention include (3β,5Z,7E)-9,10-secocholesta-5,7,10(19),16-tetraen-23-yne-3,25-diol diacetate and (3β,5Z,7E)-9,10-secocholesta-5,7,10(19),16-tetraen-23-yne-3,25-diol 3-(4-phenyl)benzoate.

The compounds of formula I can be prepared as hereinafter described in Reaction Schemes I-IV.

## SCHEME I

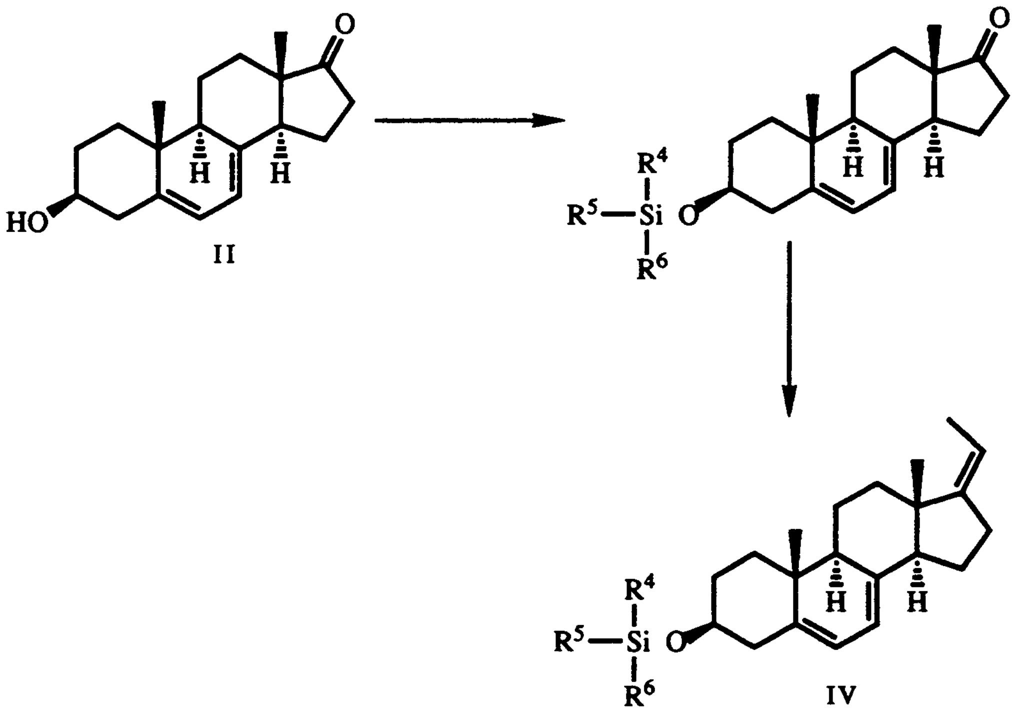

II

III

IV

wherein $R^4$, $R^5$ and $R^6$ are independently $C_1$-$C_6$ alkyl or phenyl.

In Scheme I, the compound of formula II, a known compound, is converted to a compound of formula III, by reaction with, for example, trialkylsilyl chloride such as chloro-dimethylthexylsilane in an aprotic organic solvent such as, methylene chloride in the presence of a base such as imidazole. A compound of formula III is reacted with ethylene-triphenylphosphorane in an aprotic organic solvent such as, toluene to yield a corresponding compound of formula IV.

## SCHEME II

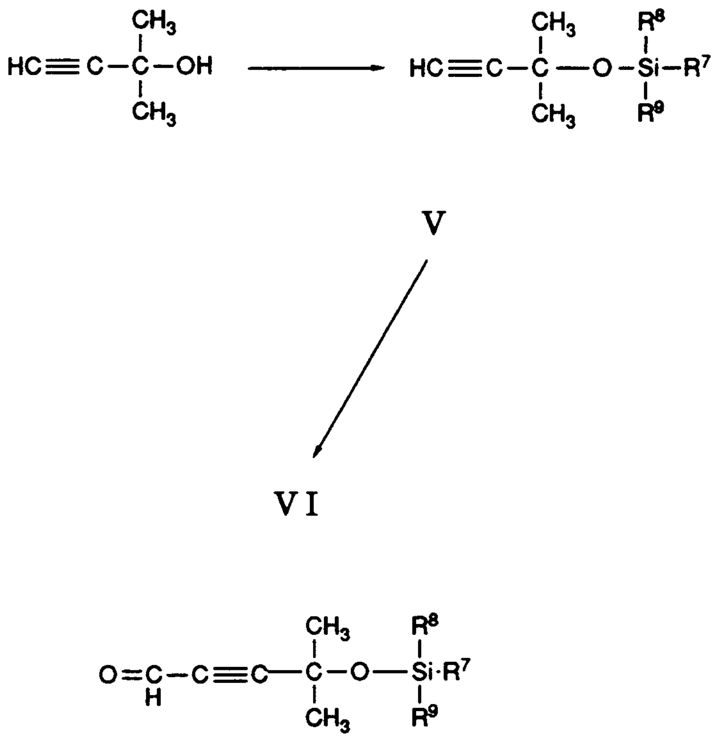

V

VI

wherein $R^7$, $R^8$, and $R^9$ are independently $C_1$-$C_6$ alkyl or phenyl.

In Scheme II, 3-hydroxy-3-methylbutyne, a known compound, is converted to a compound of formula V. e.g. by reaction with tert.butyl-dimethylsilyl chloride in a mixture of N,N-dimethylformamide and imidazole. The compound V is converted to the corresponding compound VI by reaction with N-butyllithium and N,N-dimethylformamide in anhydrous THF.

## Scheme III

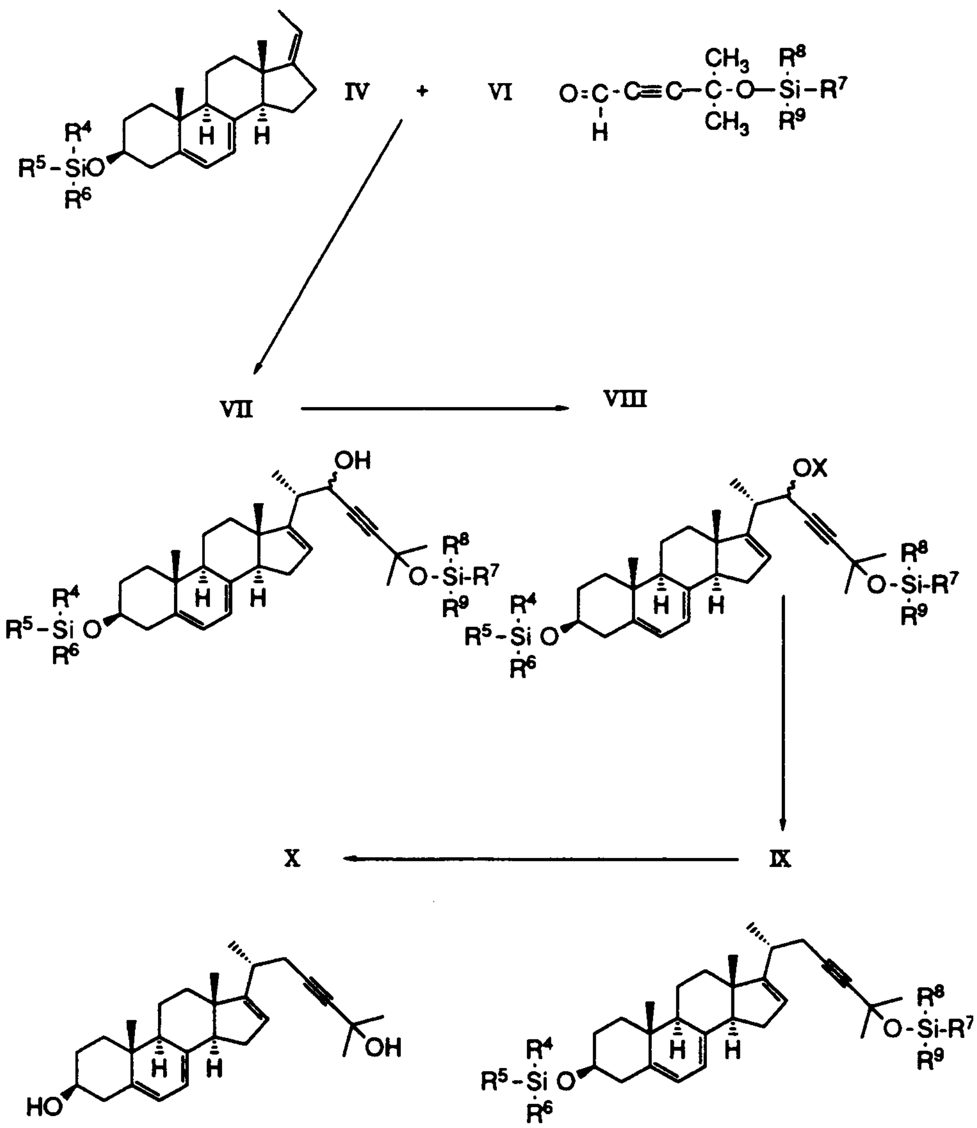

IV + VI

VII → VIII

X ← IX

wherein $R^4$, $R^5$, $R^6$, $R^7$ $R^8$, $R^9$, are independently $C_1$-$C_6$ alkyl or phenyl, X is $C(S)R^{10}$, wherein $R^{10}$ is imidazol-1-yl, -NHPh,-OPh, $-N(CH_3)_2$, or $-SCH_3$.

In Scheme III, a compound IV is converted to a corresponding compound VII, by reaction with a compound VI in an aprotic organic solvent, such as, hexane in the presence of a Lewis acid, such as dimethylaluminium chloride. The compound VII is converted to a compound VIII, by reaction with, for example, 1,1'-thiocarbonyldiimidazole, phenyl chlorothionoformate, dimethylthiocarbamoyl chloride, carbon disulfide, or preferably phenyl isothiocyanate in an aprotic solvent, such as THF in the presence of a base, such as sodium hydride. The compound VIII is converted to a corresponding compound IX, by reaction with, for example, tributyltin hydride in an aprotic organic solvent, such as hexane in the presence of a radical initiator such as 2,2'-azobis(2-methylpropionitrile). The compound IX is reacted with a fluoride salt, such as tetrabutylammonium fluoride in THF to give the compound X.

## SCHEME IV

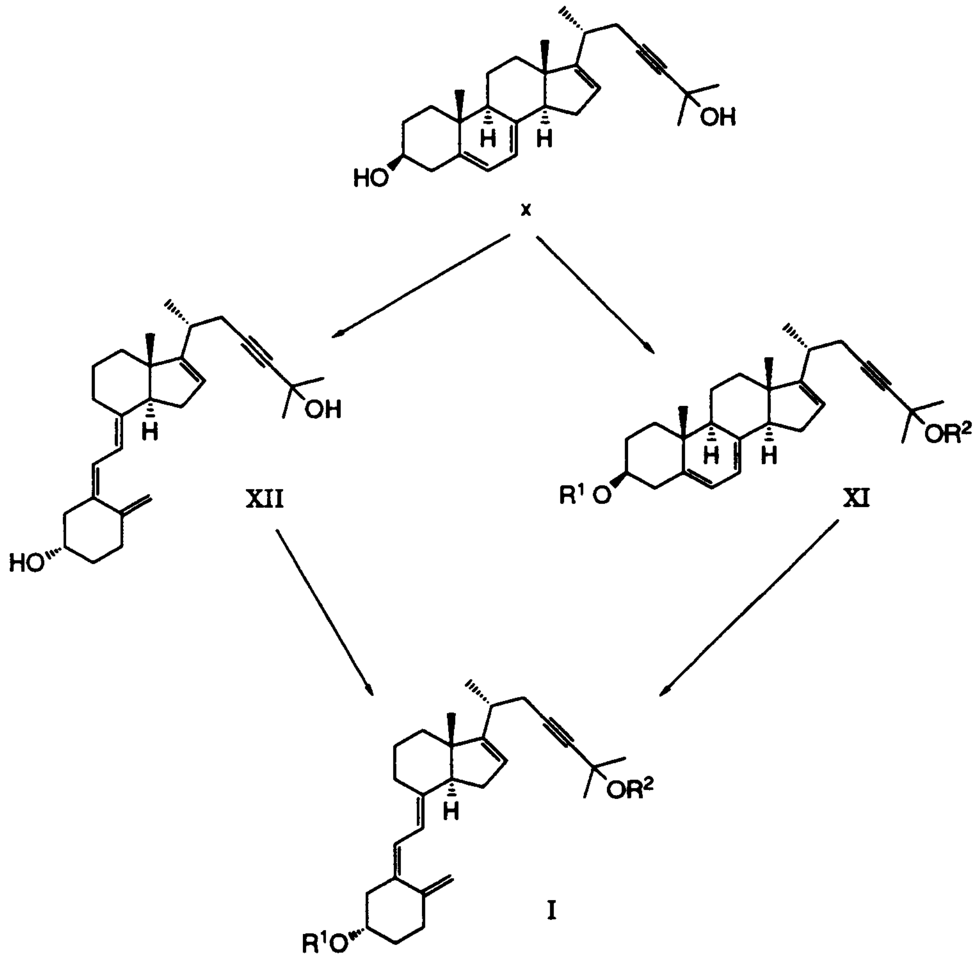

X

XII

XI

I

wherein $R^1$ and $R^2$ are independently hydrogen or acyl.

The compound of formula I may be obtained by subjecting the compound of formula X to acylation to give a compound of formula XI, which is then subjected to photolysis and thermal isomerization. This photolysis is carried out preferably by using a medium pressure mercury lamp in the presence of a 4-dialkylaminobenzoate, such as ethyl 4-dimethylaminobenzoate as a filter to block approximately 290-320 nm light. Alternatively, the compound of formula I is obtained by subjecting the compound X to the above photolysis and thermal isomerization to give a compound XII, which is acylated with, for example, 4-phenylbenzoyl chloride or acetic anhydride in an aprotic solvent, such as methylene chloride in the presence of a base, such as for exemple, triethylamine.

The compounds of formula I can be administered orally, for the treatment of hyperproliferative skin diseases such as psoriasis, basal cell carcinomas, disorders of keratinization, and keratosis, to warmblooded animals which need such treatment. More specifically, they can be administered orally to an adult human in dosages that are in the range of about 0.1 to 1000 µg per day, preferably about 7 to 70 µg per day for such treatment. They can be administered orally for the treatment of acne in humans at a dosage of about 0.7 to 700 µg per day, preferably 7 to 70 µg per day.

The compounds of formula I can be administered topically, for the treatment of hyperproliferative skin diseases such as psoriasis, basal cell carcinomas, disorders of keratinization, and keratosis, or for the treatment of sebaceous gland diseases such as acne or seborrheic dermatitis, to warmblooded animals which need such treatment. More specifically, they can be administered topically in dosages that are in the range of about 1 to about 1000 µg per gram of topical formulation per day, for such treatment.

The useful activity of compounds of formula I as agents for the treatment of hyperproliferative skin diseases can be demonstrated by the following human keratinocyte antiproliferation assay: Cell cultures were obtained from neonatal foreskin epithelial keratinocytes using standard procedures. The cells were plated at 25.000 cells/well on 6-well cluster

plates. After twenty-four hours, the cells are fed with keratinocyte growth medium supplemented to 1.5 mM $CaCl_2$ that contains test compound or vehicle. Solutions of test compounds in ethanol were prepared. Compounds were tested at four concentration in triplicate wells. Control wells were supplemented with vehicle alone at the highest concentration such as 0.1% ethanol. At the termination of the experiment, the cells were counted on an electronic particle counter, which was periodically calibrated for the correct size of keratinocytes. The number of cells was calculated according to dilution factors used and results are presented as percent inhibition from cell numbers obtained in control cultures. The $ED_{50}$ (effective dose to inhibit proliferation of 50% of the cells) for (3β,5Z,7E)-9,10-secocholesta-5,7,10(19),16-tetraen-23-yne-3,25-diol diacetate was 0.3 μM.

1α,25-dihydroxycholecalciferol is disclosed in Journal of Investigative Dermatology 92, (1989) 475 as an agent for reducing the size of sebaceous glands in the ears of male Syrian hamsters. The useful activity of compounds of formula I as agents for the treatment of sebaceous gland diseases such as acne or seborrheic dermatitis can be demonstrated by evaluating their effect on the sebaceous glands of the hamster ear after oral administration of the compounds. 200 μl of a compound of the invention was dissolved in propylene glycol, administered daily (5 days per week) to male Syrian hamster. The animals were sacrificed after 4 weeks and the ears were processed for histological evaluation. The area of the sebaceous glands was measured on histologically prepared cross sections of the ear by image analysis. The data obtained from this study is presented in the table below:

| Compound | Dose μmoles/kg/day | % Change in Hamster Ear Sebaceous Gland Size Cross Section Analysis |
|---|---|---|
| (3β,5Z,7E)-9,10-Secocholesta-5,7,10(19),16-tetraen-23-yne-3,25-diol 3-(4-phenyl)benzoate | 0.105 | -20 |
| | 1.05 | -27 |
| | 10.5 | -31 |
| | 105 | -40 |
| (3β,5Z,7E)-9,10-Secocholesta-5,7,10(19),16-tetraen-23-yne-3,25-diol diacetate | 0.150 | -18 |
| | 1.5 | -28 |
| | 15 | -37 |
| | 150 | -42 |

The above data demonstrate that compounds of the invention are useful as agents in the treatment of sebaceous gland diseases such as acne or seborrheic dermatitis.

Oral dosage forms comprising compounds of formula I, may be incorporated in capsules, tablets and the like with pharmaceutically acceptable carrier materials. Illustrative of such carrier materials are a binder such as gum tragacanth, acacia, corn starch, or gelatin; an excipient such as dicalcium phosphate; a disintegrating agent such as corn starch, potato starch and algenic acid; a lubricant such as magnesium stearate, a sweetening agent such as sucrose, lactose, or saccharin; a flavoring agent such as peppermint, oil of wintergreen or cherry. Various other materials may be present as coating or to otherwise modify the physical form of the dosage unit. For instance, tablets may be coated with shellac, sugar, or both. A syrup or elixir may contain the active compound, sucrose as a sweetening agent, methyl and propyl parabens as preservatives, a dye, and a flavoring such as cherry or orange flavor.

Topical dosage forms comprising compounds of formula I include ointments and creams encompassing formulations having oleaginous, adsorbable, water-soluble and emulsion-type bases such as petrolatum, lanolin and polyethylene glycols. Lotions are liquid preparations and vary from simple solutions to aqueous or hydroalcoholic preparations containing finely divided substances. Lotions can contain suspending or dispersing agents, for example, cellulose derivatives such as ethyl cellulose and methyl cellulose; gelatin or gums, which incorporate the active ingredient in a vehicle made up of water, alcohol or glycerin. Gels are semi-solid preparations made by gelling a solution or suspension of the active ingredient in a carrier vehicle. The vehicles, which can be hydrous or anhydrous, are gelled using e.g., carboxy polymethylene, and neutralized to a proper gel consistency with the use of alkalies, such as, sodium hydroxide and amines, such as, polyethylenecocoamine. As used herein, the term "topical" denotes the use of the active ingredient, incorporated in a suitable pharmaceutical carrier, and applied at the site of the inflammation for the exertion of local action. Accordingly, the topical compositions include those pharmaceutical forms in which the compound is applied externally by direct contact with the skin. The topical dosage forms comprise gels, creams, lotions, ointments, powders, aerosols and other conventional forms for applying medication to the skin obtained by admixing the compounds of formula I with known pharmaceutical topical carrier materials. In addition to application to the skin, the topical compositions of this invention can also be employed in the treatment of inflammations of mucous membranes, where such

membranes are accessible to topical application of medication. For example, the topical composition can be applied to the mucous lining of the mouth or lower colon.

The examples which follow further illustrate the invention.

Example 1

After a mixture of 149.9 g (3β)-3-hydroxyandrosta-5,7-dien-17-one and 56.9 g imidazole in 500 ml of dichloromethane was cooled to 3°C, 144 ml dimethylthexylsilyl chloride was added dropwise keeping the temperature below 6°C. After stirring at room temperatur overnight, the mixture was washed with water, and the aqueous layer was extracted with dichloromethane. The organic layers were washed with saturated aqueous $NaHCO_3$, dried and concentrated. The resulting solid was suspended in 700 ml of methanol containing 14 ml of triethylamine, and the suspension was refluxed for 20 minutes. After cooling, the precipitate was filtered, washed with methanol/$H_2O$ (9:1), and dried to give 185.1 g of (3β)-3-[[(1,1,2-trimethylpropyl)dimethylsilyl]oxy]androsta-5,7-dien-17-one, mp 119-125°C.

Example 2

A mixture of 240.2 g (ethyl)triphenylphosphonium bromide, 72.5 g potassium t-butoxide, and 1 l toluene was stirred for 1 hr. Then, 185 g of the product of Example 1 was added with 50 ml of toluene. The temperature was kept below 25°C. After stirring overnight, the reaction was quenched with 24.5 ml of acetic acid. After stirring, the solid was removed by filtration and washed with toluene. The filtrate and washes were concentrated, 200 ml of methanol was added and the mixture was concentrated again. The residue was dissolved in a mixture of 650 ml methanol, 65 ml water and 650 ml hexane. The methanol/water layer was extracted with 325 ml of hexane. The combined hexane layers were concentrated. Then, 200 ml of methanol was added and the mixture was concentrated again. The resulting solid was suspended in 800 ml of methanol containing 8 ml of triethylamine, and the suspension was refluxed. After cooling overnight, the precipitate was filtered, washed with methanol, and dried to give 178.1 g of (3β,17Z)-(1,1,2-trimethyl-propyl)(pregna-5,7,17(20)-trien-3-yloxy)dimethylsilane, mp 94-97°C, $[\alpha]_D$ -62.8° (c 0.94, ethanol).

Example 3

a) To a solution of 50.0 g (1,1-dimethylethyl)[(1,1-dimethyl-2-propynyl)oxy]dimethylsilane in 200 ml of THF at -70°C was added 112 ml of 2.5M butyl lithium in hexane dropwise, keeping the temperature below -55°C. The mixture was stirred, then 50 ml of DMF was added dropwise. The reaction was then quenched by the addition of 32 ml of acetic acid. After the mixture was allowed to warm to -20°C, 200 ml of hexane and 200 ml of water were added. The aqueous layer was extracted with hexane. The combined organic layers were washed with saturated aqueous $NH_4Cl$ solution and then with brine. After drying, the solution was concentrated to dryness. Then the residue was distilled to give 48.8 g of 4-[[(1,1-dimethylethyl)dimethylsilyl]oxy]-4-methyl-2-pentynal: bp 50°C (0.5 mm Hg).

b) A mixture of 57.65 g ((3β,17Z)-(1,1,2-trimethylpropyl)(pregna-5,7,17(20)-trien-3-yloxy)dimethylsilane, 34.11 g of the product of Example 3a), and 800 ml hexane was cooled to about -40°C. Then, 185 ml 1M dimethylaluminium chloride in hexane was added dropwise. After stirring at -40°C, 400 ml of 5% aqueous sodium phosphate dibasic (w/v) was added dropwise, and the mixture was allowed to warm to 5°C. Then, 300 ml of 3N HCl was added dropwise at 0-5°C followed by 40 g of kieselguhr. The solid was filtered and wahed with hexane. The aqueous layer was extracted with hexane. The combined hexane solutions were washed successively with water, 10% aqueous $NaHCO_3$, and brine. The hexane layer was dried and concentrated to give 90 g of crude (3β)-25-[[(1,1-dimethylethyl)dimethylsilyl]oxy]-3-[[dimethyl(1,1,2-trimethylpropyl)silyl]-oxy]cholesta-5,7,16-trien-23-yn-22-ol (5:1 mixture of epimers at C-22), which was used for the next step.

Example 4

Sodium hydride (60% dispersion, 6.55 g) was suspended in 200 ml of THF followed by the addition of 446 mg imidazole at 5°C. To this suspension, a solution of crude 93 g (3β)-25-[[(1,1-dimethylethyl)dimethylsilyl]oxy]-3 [[dimethyl(1,1,2-trimethylpropyl)silyl]oxy)cholesta-5,7,16-trien-23-yn-22-ol in 700 ml of THF was added dropwise at 8-10°C. The resulting suspension was stirred, then, a solution of 17.3 ml phenyl isothiocyanate in THF was added, and the mixture was stirred at 10°C. The reaction was quenched by the dropwise addition of 500 ml of 10% aqueous $NaHCO_3$ followed by 70 g kieselguhr. After stirring, the solid was filtered and washed with ethyl acetate. The aqueous layer was extracted with ethyl acetate. The combined organic layers were washed successively with 10% aqueous $NaHCO_3$ and brine, dried, and concentrated to dryness. Some impurities were removed by dissolving 132 g of the residue in 3% ethyl acetate in hexane and filtering it through silica gel. The later was washed with 3% ethyl acetate in hexane. The eluate was collected and concentrated to dryness to give 117 g of crude phenylcarbamothioic acid O-[(3β)-25-[[(1,1-dimethylethyl)dimethylsilyl]oxy]-3-[[dimethyl(1,1,2-trimethylpropyl)silyl]oxy]cholesta-5,7,16-trien-23-yn-22-yl] ester, which was

used for the next step.

Example 5

To a mixture of 70.5 ml tributyltin hydride and 1.08 g 2,2'-azobis(2-methylpropionitrile) AIBN) in 50 ml of hexane at 80°C was added a solution of 117 g of the product of Example 4 in 1.5 l of hexane. After refluxing 35 ml of tributyltin hydride and AIBN (1.07 g) were added. After refluxing, the mixture was cooled to room temperature and stirred. The precipitate was filtered, and the filtrate was concentrated. The residue was dissolved in hexane and filtered through silica gel. The solumn was eluted with hexane and then with 20% toluene in hexane. The fractions containing the product were combined and concentrated to give 114 g of crude [[(3β)-25-[[(1,1-dimethylethyl)dimethyl-silyl]oxy]cholesta-5,7,16-trien-23-yn-3-yl]oxy]dimethyl-(1,1,2-trimethylpropyl)-silane which was used in the next step. An analytical sample was obtained by chromatographic purification followed by crystallization from ethyl acetate/methanol (1:1): mp 62-66°C.

Example 6

To a solution of 114 g of the product of Example 5 in 500 ml of THF was added 200 g tetrabutylammonium fluoride hydrate with 500 ml of THF. After stirring, the mixture was diluted with 500 ml of ethyl acetate and 500 ml of 10% aqueous ammonium chloride. The aqueous layer was extracted with ethyl acetate. The organic layers were washed with 10% aqueous ammonium chloride, water, and brine. The organic layer was dried and concentrated. The resulting suspension was stirred and then stored in a freezer overnight. The solid was filtered, washed with ethyl acetate, and dried. The product was suspended in methanol and the mixture was refluxed. Then, water was added dropwise. The suspension was stirred at 60°C, and stored in a refrigerator overnight. The precipitate was filtered, washed with 50% aqueous methanol, and dried at 45°C to give 25,4 g of (3β)-cholesta-5,7,16-trien-23-yne-3,25-diol, mp 185-192°C.

Example 7

To a cold suspension of 75.3 g (3β)-cholesta-5,7,16-trien-23-yn-3,25-diol in 450 ml of dichloromethane was added 107 ml acetic anhydride and 160 ml triethylamine. After the suspension was cooled to 2°C, 4.6 g 4-dimethylaminopyridine was added. The cold bath was removed and the mixture was stirred at room temperature overnight. Alter cooling again with an ice-water bath, 30.7 ml methanol was added. and the mixture was stirred at room temperature. The mixture was washed with water and the aqueous layer was extracted with dichloromethane. The combined organic layers were washed with IN HCl, and the aqueous layer was extracted with dichloromethane. The combined organic layers were washed with saturated aqueous $NaHCO_3$, dried and concentrated to dryness. The residue was dissolved in 95% methanol. After a seed crystal was added, the suspension was stored in a refrigerator. The precipitate was filtered and washed with 90% methanol. After drying, 82.05 g of (3β)-cholesta-5,7,16-trien-23-yne-3,25-diol diacetate was obtained, mp 98-101°C.

Example 8

A solution of 16.4 g (3β)-cholesta-5,7,16-trien-23-yne-3,25-diol diacetate and 1.64 g ethyl 4-dimethylaminobenzoate in 1.7 l of tert.-butyl methyl ether at -20°C was irradiated with a 450 W medium pressure lamp through a quartz immersion well. During the photolysis, the arc housing was constantly purged with a slow current of nitrogen. After 8 hr of irradiation at 0 to -20°C, a uranium filter was inserted in the arc housing, and then 66 mg of 9-acetylanthracene was added to the solution. After 1 hr 45 min of irradiation through the filter at 0 to -20°C, the solution was allowed to warm to room temperature overnight, and then washed with 3N HCl. The organic layer was washed with saturated aqueous $NaHCO_3$ and dried. The solution was concentrated to dryness. The residual oil was purified by chromatography on silica gel, eluting with 3 l of 7% ethyl acetate in hexane. The desired fraction were combined and concentrated to give 13 g of a clear oil. A total of five such experiments produced 65 g of the crude photo-product from 84 g of (3β)-cholesta-5,7,16-trien-23-yne-3,25-diol diacetate. A solution of the crude photo-products in ethyl acetate was refluxed for 4 hr and then allowed to cool to room temperature. The solution was concentrated. The residual semi-solid was dissolved in methanol. The solution was cooled to room temperature and then stored in a refrigerator overnight. The crystalline material was filtered and washed with 95% methanol. The crystalline solid was dried to afford 33.0 g (3β,5Z,7E)-9,10-secocholesta-5,7,10(19),16-tetraen-23-yne-3,25-diol diacetate, mp 94-95°C.

Example 9

A solution of 18.0 g (3β)-cholesta-5,7,16-trien-23-yne-3,25-diol, 3.6 g ethyl dimethylaminobenzoate, and 1.7 l ethanol at -20°C was irradiated with a 450 watt medium pressure lamp through a quatz immersion well for 7 hr. A uranium

filter was inserted in the arc housing, and then 180 mg of 9-acetylanthracene was added to the solution. After 2 hr. of irradiation through the filter at 0 to -20°C, the solution was allowed to warm to room temperature overnight. The solvent was removed under reduced pressure. The residual ethanol was removed by co-evaporation with toluene. Then, the residue was suspended in toluene. After dilution with hexane, the suspension was stored in a refrigerator. The precipitate was filtered and washed with toluene/hexane (1:1) and then with toluene. The combined filtrate and washes were concentrated. The obtained solution was stirred at 90-100°C and then allowed to cool to room temperature. The solution was concentrated to dryness and then purified by chromatography on silica gel eluting with 5-10% $CH_3CN$ in $CH_2Cl_2$ to give 8.35 g (3β,5Z,7E)-9,10-secocholesta-5,7,10(19),16-tetraen-23-yne-3,25-diol, which was used in the next step without further purification.

Example 10

To an ice-cold solution of 8.35 g (3β,5Z,7E)-9,10-secocholesta-5,7,10(19),16-tetraen-23-yne-3,25-diol, 513 mg 4-dimethylaminopyridine, and 5.3 ml triethylamine in dichloromethane was added 5.99 g (4-phenyl)benzoyl chloride. After stirring at room temperature, the mixture was diluted with dichloromethane and washed with water. The aqueous layer was extracted with dichloromethane. The combined dichloromethane solutions were washed with saturated aqueous $NaHCO_3$ solution. Each aqueous layer was extracted with dichloromethane. The combined organic layers were dried and concentrated. The residue was purified by chromatography on silica gel. The product was dissolved in methanol, and the solution was cooled to room temperature with stirring. Then the suspension was kept in a refrigerator. The precipitate was filtered and washed with 95% methanol. The solid was dried to give 5.73 g of (3β,5Z,7E)-9,10-secocholesta-5,7,10(19),16-tetraen-23-yne-3,25-diol 3-(4-phenyl)benzoate, mp 105-110°C.

Example 11

To the stirred mixture of 25 g of 3-hydroxy-3-methyl-butyne, 50 ml of anhydrous DMF and 44.5 g imidazole cooled in an ice-bath was added 50 g tert.butyldimethylsilyl chloride. Stirring in the ice-bath was continued and then at room temperature overnight. After addition of 250 mg of dimethylaminopyridine, the reaction mixture was heated at 70°C and then poured into cold water. It was then extracted with ether. The organic extract was washed with brine, dried and evaporated to dryness. The product was purified by distillation yielding 3-(tert.butyldimethylsilyl)oxy-3-methyl-butyne. To the solution of 10 g of 3-(tert.butyldimethylsilyl)oxy-3-methyl-butyne in 25 ml of THF cooled at -78°C in an argon atmosphere, was added dropwise 40 ml 1.6M solution of N-butyllithium in hexane, which was followed by addition of 31 ml of DMF. The reaction mixture was stirred at -78°C, and then quenched by addition of ice and pouring into brine. It was then extracted with pentane. The pentane extract was washed with saturated ammonium chloride solution, water and brine, dried and evaporated to dryness. The product was purified by distillation to give 8.88 g of 4-(tert.butyldimethylsilyl)oxy-4-methyl-pentynal.

The following galenical formulations were prepared in a manner known per se:

Example A

| Wet granulation formulation: | | | | |
|---|---|---|---|---|
| Ingredients | mg/tablet | | | |
| (3β,5Z,7E)-9,10-secocholesta-5,7,10(19),16-tetraen-23-yne-3,25-diol [diacetate or 3-(4-phenyl)benzoate] | 0.1 | 0.5 | 5.0 | 5.0 |
| Anhydrous lactose | 106.9 | 106.5 | 102.0 | 118.0 |
| Microcrystalline cellulose | 15.0 | 15.0 | 15.0 | 25.0 |
| Modified starch | 7.0 | 7.0 | 7.0 | 10.0 |
| Magnesium stearate | 1.0 | 1.0 | 1.0 | 2.0 |
| | 130.0 | 130.0 | 130.0 | 160.0 |

Example B

| Capsule formulation: | | | | |
|---|---|---|---|---|
| Ingredients | mg/capsule | | | |
| (3β,5Z,7E)-9,10-secocholesta-5,7,10(19),16-tetraen-23-yne-3,25-diol [diacetate or 3-(4-phenyl)benzoate] | 0.1 | 0.5 | 5.0 | 25.0 |
| Hydrous lactose | 168.9 | 168.5 | 159.0 | 123.0 |
| Corn starch | 20.0 | 20.0 | 25.0 | 35.0 |
| Talc | 10.0 | 10.0 | 10.0 | 15.0 |
| Magnesium stearate | 1.0 | 1.0 | 1.0 | 2.0 |
| | 200.0 | 200.0 | 200.0 | 200.0 |

Example C

| Tablet formulation (wet granulation): | | | |
|---|---|---|---|
| Ingredients | mg/tablet | | |
| (3β,5Z,7E)-9,10-secocholesta-5,7,10(19),16-tetraen-23-yne-3,25-diol [diacetate or 3-(4-phenyl)benzoate] | 25 | 100 | 500 |
| Anhydrous lactose | 105 | 30 | 150 |
| Pregelatinized starch | 6 | 6 | 30 |
| Microcrystalline cellulose | 30 | 30 | 150 |
| Magnesium stearate | 1 | 1 | 5 |
| | 167 | 167 | 835 |

Example D

| Capsule formulation: | | | |
|---|---|---|---|
| Ingredients | mg/capsule | | |
| (3β,5Z,7E)-9,10-secocholesta-5,7,10(19),16-tetraen-23-yne-3,25-diol [diacetate or 3-(4-phenyl)benzoate] | 25 | 100 | 500 |
| Pregelatinized corn starch | 83 | 8 | 40 |
| Modified starch | 4 | 4 | 20 |
| Talc | 4 | 4 | 20 |
| Magnesium stearate | 1 | 1 | 5 |
| | 117 | 117 | 585 |

## Claims

1. A compound of the formula

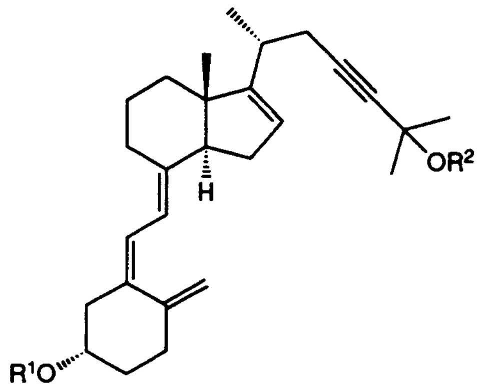

I

wherein $R^1$ and $R^2$ are hydrogen or acyl; provided that only one of $R^1$ or $R^2$ is hydrogen.

2. The compound of claim 1, wherein $R^1$ is $R^3CO$, $R^3$ is lower alkyl or aryl and $R^2$ is hydrogen or acetyl.

3. The compound of claim 1, selected from the group consisting of (3β,5Z,7E)-9,10-secocholesta-5,7,10(19),16-tetraen-23-yne-3,25-diol diacetate and (3β,5Z,7E)-9,10-secocholesta-5,7,10(19),16-tetraen-23-yne-3,25-diol 3-(4-phenyl)benzoate.

4. A compound of the formula

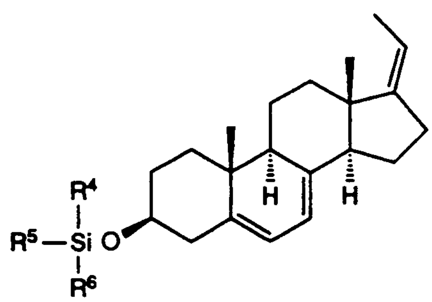

IV

wherein $R^4$, $R^5$ and $R^6$ are independently $C_1$-$C_6$ alkyl or phenyl, particularly wherein $R^4$ and $R^5$ are methyl and $R^6$ is thexyl;

or of the formula

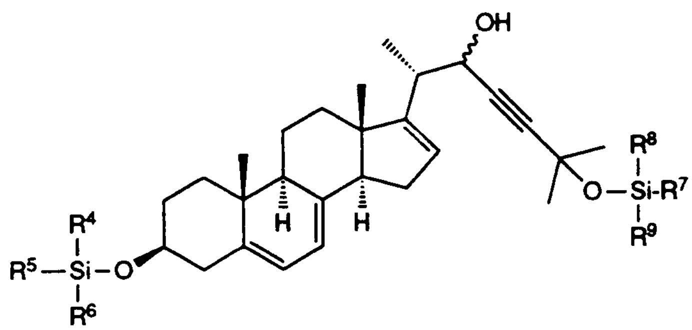

VII

wherein $R^4$ to $R^9$ are independently $C_1$-$C_6$ alkyl or phenyl, particularly wherein $R^4$, $R^5$, $R^7$ and $R^8$ are methyl, $R^6$ is thexyl, $R^9$ is tert.-butyl;

or of the formula

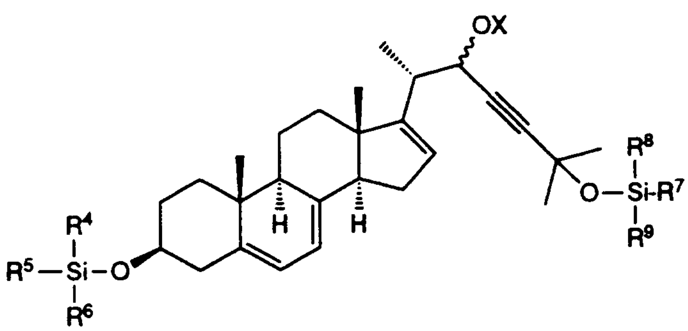

VIII

wherein $R^4$ to $R^9$ are independently $C_1$-$C_6$ alkyl or phenyl and X is -C(S)$R^{10}$, wherein $R^{10}$ is imidazol-1-yl, -NHPh, -OPh, -$N(CH_3)_2$, or -$SCH_3$, particularly wherein $R^4$, $R^5$, $R^7$ and $R^8$ are methyl, $R^6$ is thexyl, $R^9$ is tert.-butyl, $R^{10}$ is -NHPh, and Ph is phenyl, or

of the formula

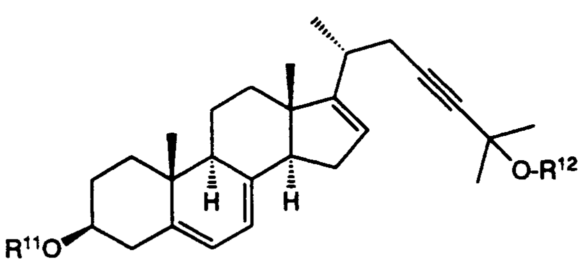

XIII

wherein $R^{11}$ and $R^{12}$ are independently -Si($R^4$,$R^5R^6$), hydrogen or acyl, $R^4$, $R^5$ and $R^6$ are independently $C_1$-$C_6$ alkyl or phenyl, particularly wherein $R^{11}$ is dimethylthexylsilyl and $R^{12}$ is tert.-butyldimethylsilyl, or wherein $R^{11}$ and $R^{12}$ are hydrogen, or wherein $R^{11}$ and $R^{12}$ are acetyl; or

of the formula

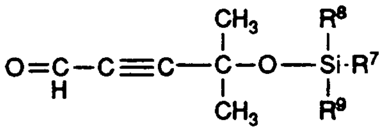

VI

wherein $R^7$, $R^8$ and $R^9$ are independently $C_1$-$C_6$ alkyl or phenyl.

5. A compound according to Claim 1, 2 or 3 for use as a therapeutically active agent, particularly for the treatment of hyperproliferative disorders of the skin, such as psoriasis, and for the treatment of sebaceous gland diseases, such as acne and seborrheic dermatitis.

6. A process for preparing a compound of the formula

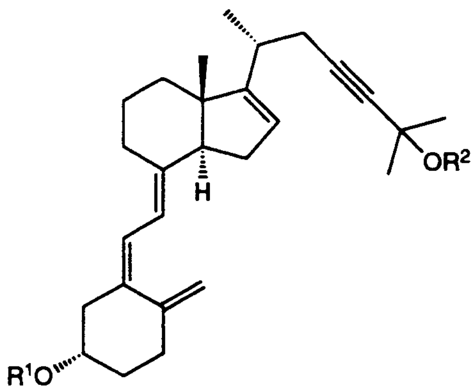

I

wherein $R^1$ and $R^2$ are hydrogen or acyl,

which comprises subjecting a compound of the formula

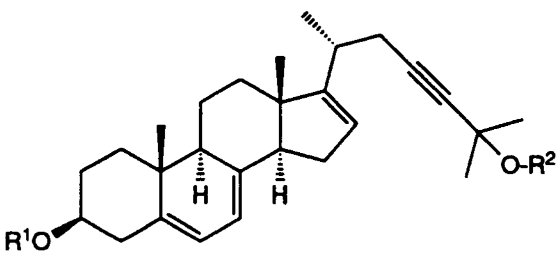

XI

wherein $R^1$ and $R^2$ are as described above,

to photolysis, preferably by irradiation with a medium pressure mercury lamp utilizing a 4-dialkylaminobenzoate, particularly ethyl 4-dimethylamino-benzoate, as a filter to block about 290-320 nm light.

7. A process for preparing a compound of the formula

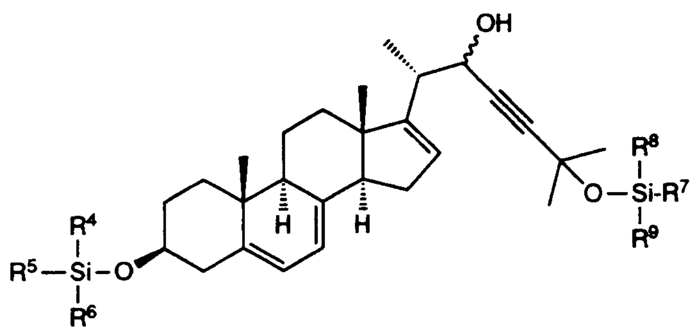

VII

wherein $R^4$, $R^5$, $R^6$, $R^7$, $R^8$ and $R^9$ are independently $C_1$-$C_6$ alkyl or phenyl,

comprising reacting a compound of the formula

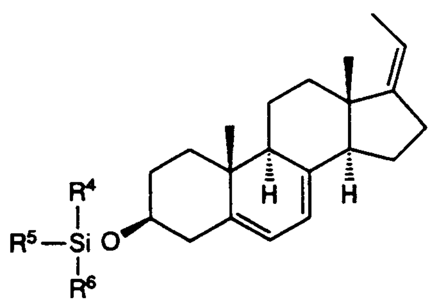

IV

wherein $R^4$, $R^5$, $R^6$ are as described above

with a compound of the formula

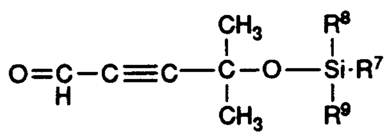

VI

wherein $R^7$, $R^8$ and $R^9$ are as described above.

**8.** A process for preparing a compound of the formula

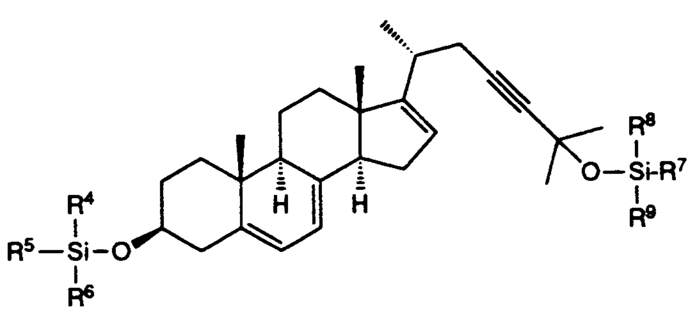

IX

wherein $R^4$, $R^5$, $R^6$, $R^7$, $R^8$ and $R^9$ are independently $C_1$-$C_6$ alkyl or phenyl,

comprising converting a compound of the formula

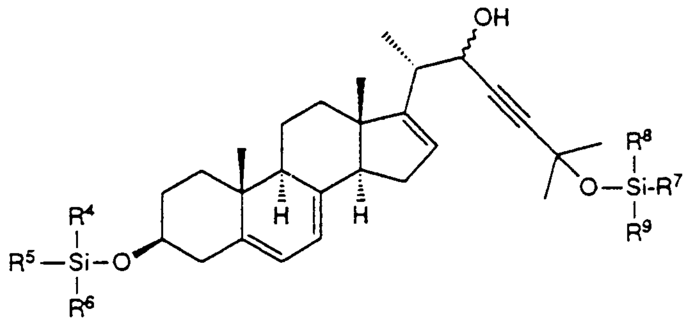

VII

wherein $R^4$, $R^5$, $R^6$, $R^7$, $R^8$ and $R^9$ are as described above,

into a compound of formula

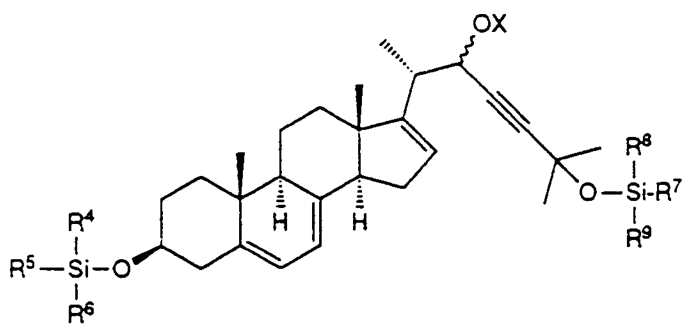

VIII

wherein $R^4$, $R^5$, $R^6$, $R^7$, $R^8$ and $R^9$ are as described above, X is -C(S)$R^{10}$, wherein $R^{10}$ is imidazol-1-yl, -NHPh,-OPh, -N$(CH_3)_2$, or -$SCH_3$. and Ph is phenyl

by reacting compound VII with 1,1'-thiocarbonyl-diimidazole, phenyl chlorothionoformate, dimethylthiocarbamoyl chloride, carbon disulfide, or phenyl isothiocyanate in an aprotic solvent, in the presence of a base, and converting the obtained compound of formula VIII into that of formula IX, by reaction with tributyltin hydride in an aprotic organic solvent, in the presence of a radical initiator .

**9.** A pharmaceutical composition, particularly for the treatment of hyperproliferative disorders of the skin, such as psoriasis, and for the treatment of sebaceous gland diseases, such as acne and seborrheic dermatitis, comprising an effective amount of a compound of the formula I according to Claim 1, and a pharmaceutically acceptable carrier.

**10.** The use of a compound as in Claim 1, 2 or 3 for the manufacture of a medicament for the treatment of hyperproliferative disorders of the skin, such as psoriasis, and for the treatment of sebaceous gland diseases, such as acne and seborrheic dermatitis.

**Patentansprüche**

**1.** Eine Verbindung der Formel

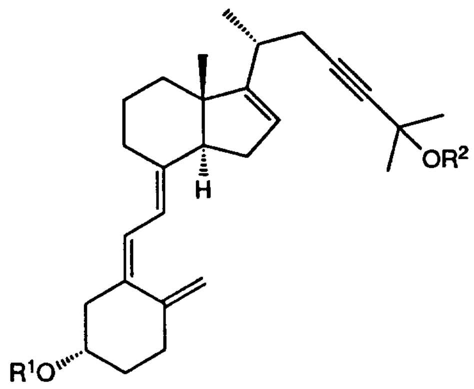

I

worin $R^1$ und $R^2$ Wasserstoff oder Acyl sind, wobei nur eins von $R^1$ und $R^2$ Wasserstoff ist.

2. Die Verbindung nach Anspruch 1, worin $R^1$ eine Gruppe $R^3CO$, $R^3$ nieder - Alkyl oder Aryl und $R^2$ Wasserstoff oder Acetyl ist.

3. Die Verbindung nach Anspruch 1 aus der Gruppe von (3β,5Z,7E)-9,10-Secocholesta-5,7,10(19),16-tetraen-23-yn-3,25-diol-diacetat und (3β,5Z,7E)-9,10-Secocholesta-5,7,10(19),16-tetraen-23-yn-3,25-diol-3-(4-phenyl)benzoat.

4. Eine Verbindung der Formel

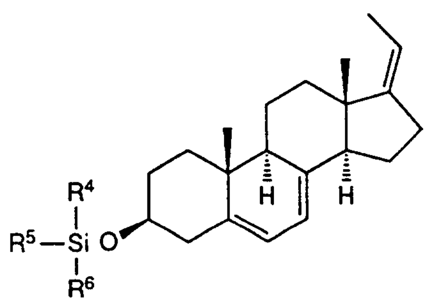

IV

worin $R^4$, $R^5$, und $R^6$ unabhängig voneinander $C_1$-$C_6$-Alkyl oder Phenyl, insbesondere worin $R^4$ und $R^5$ Methyl und $R^6$ Thexyl sind;
oder der Formel

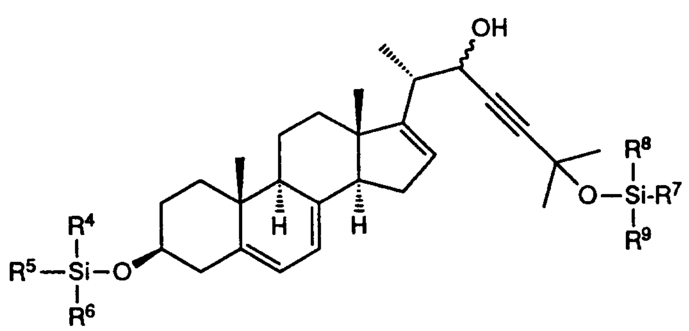

VII

worin $R^4$ bis $R^9$ unabhängig voneinander $C_1$-$C_6$-Alkyl oder Phenyl, insbesondere worin $R^4$, $R^5$, $R^7$ und $R^8$ Methyl, $R^6$ Thexyl, $R^9$ tert-Butyl sind;
oder der Formel

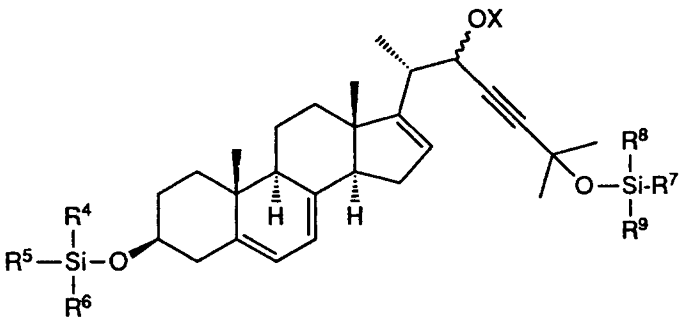

VIII

worin $R^4$ bis $R^9$ unabhängig voneinander $C_1$-$C_6$-Alkyl oder Phenyl und X eine Gruppe -C(S)$R^{10}$, worin $R^{10}$ Imidazol-1-yl, -NHPh, -OPh, -N$(CH_3)_2$ oder -$SCH_3$, insbesondere worin $R^4$, $R^5$, $R^7$ und $R^8$ Methyl, $R^6$ Thexyl, $R^9$ tert-Butyl, $R^{10}$ die Gruppe -NHPh und Ph Phenyl sind,
oder der Formel

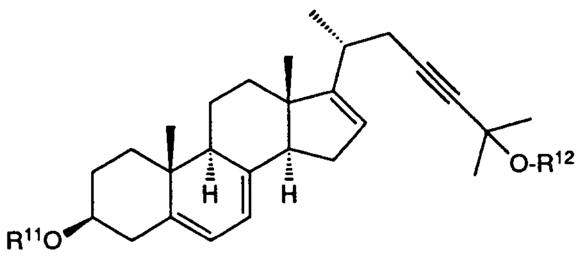

XIII

worin $R^{11}$ und $R^{12}$ unabhängig voneinander -Si ($R^4$,$R^5$,$R^6$), Wasserstoff oder Acyl, $R^4$, $R^5$ und $R^6$ unabhängig voneinander $C_1$-$C_6$-Alkyl oder Phenyl, insbesondere worin $R^{11}$ Dimethylthexylsilyl und $R^{12}$ tert-Bytyldimethylsilyl, oder worin $R^{11}$ und $R^{12}$ Wasserstoff oder worin $R^{11}$ und $R^{12}$ Acetyl sind,
oder der Formel

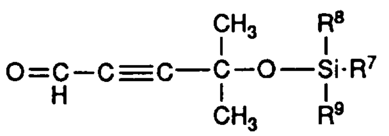

VI

worin $R^7$, $R^8$ und $R^9$ unabhängig voneinander $C_1$-$C_6$-Alkyl oder Phenyl sind.

5. Eine Verbindung nach Anspruch 1, 2 oder 3 zur Verwendung als therapeutisch wirksames Mittel, insbesondere für die Behandlung von hyperprofilerativen Hauterkrankungen wie Psoriasis, und für die Behandlung von Krankheiten der Talgdrüsen, wie Akne und seborrhoische Dermatitis.

6. Verfahren zur Herstellung einer Verbindung der Formel

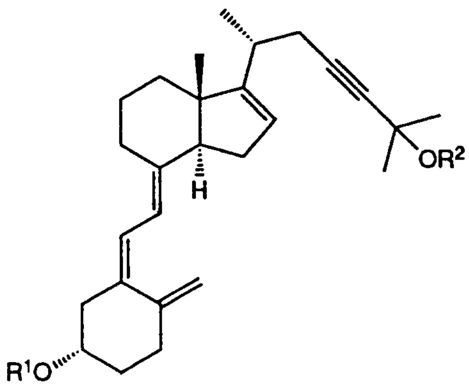

I

worin $R^1$ und $R^2$ Wasserstoff oder Acyl sind, dadurch gekennzeichnet, dass man eine Verbindung der Formel

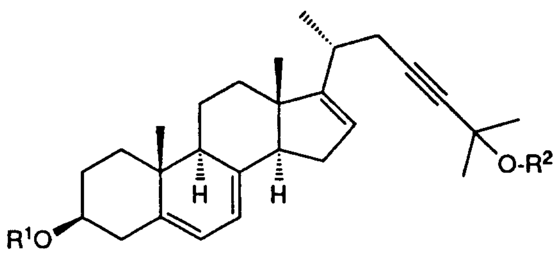

XI

worin $R^1$ und $R^2$ obige Bedeutung haben, einer Photolyse unterwirft, vorzugsweise durch Bestrahlung mit einer Mitteldruck-Quecksilberlampe, unter Verwendung eines 4-Dialkylaminobenzoats, insbesondere des 4-Dimethylaminobenzoats, als Filter zur Blockierung des Lichts von ungefähr 290 - 320 nm.

**7.** Verfahren zur Herstellung einer Verbindung der Formel

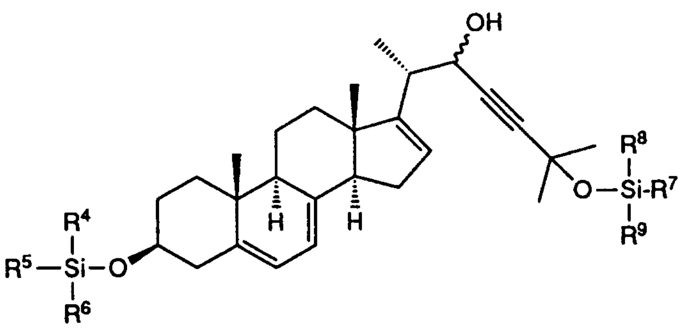

VII

worin $R^4$, $R^5$, $R^6$, $R^7$, $R^8$ und $R^9$ unabhängig voneinander $C_1$-$C_6$-Alkyl oder Phenyl sind, dadurch gekennzeichnet, dass man eine Verbindung der Formel

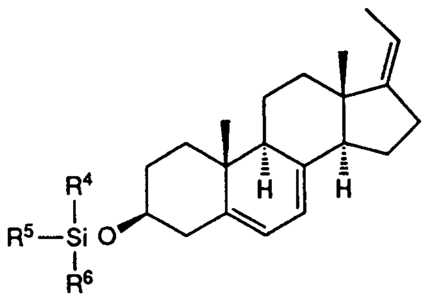

IV

worin $R^4$, $R^5$ und $R^6$ obige Bedeutung haben, mit einer Verbindung der Formel

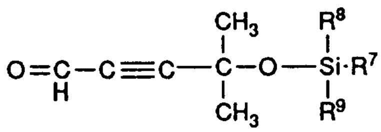

VI

worin $R^7$, $R^8$ und $R^9$ obige Bedeutung haben,
umsetzt.

**8.** Verfahren zur Herstellung einer Verbindung der Formel

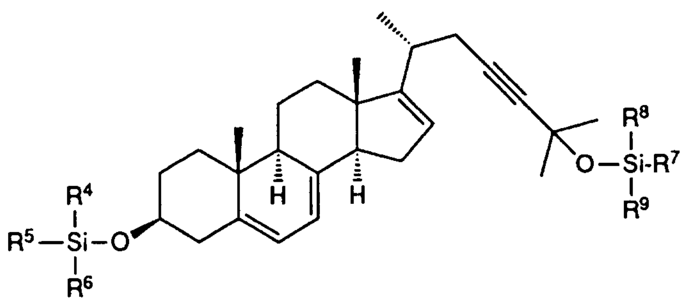

IX

worin $R^4$, $R^5$, $R^6$, $R^7$, $R^8$ und $R^9$ unabhängig voneinander $C_1$-$C_6$-Alkyl oder Phenyl sind, dadurch gekennzeichnet, dass man eine Verbindung der Formel

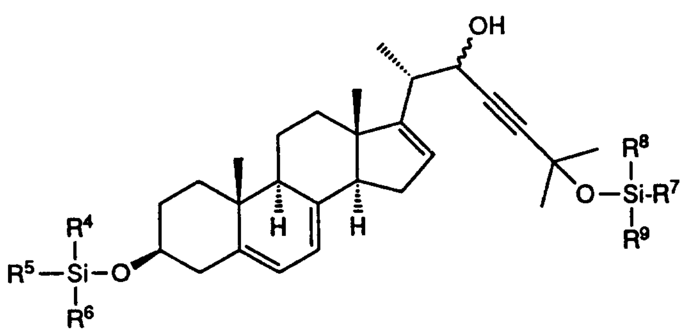

VII

worin $R^4$, $R^5$, $R^6$, $R^7$, $R^8$ und $R^9$ obige Bedeutung haben,
in eine Verbindung der Formel

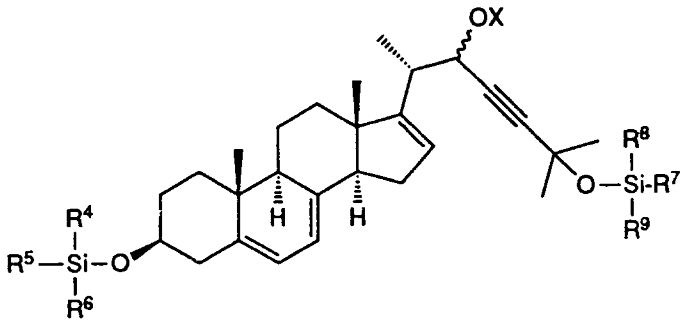

VIII

worin $R^4$, $R^5$, $R^6$, $R^7$, $R^8$ und $R^9$ obige Bedeutung haben, X eine Gruppe $-C(S)R^{10}$, worin $R^{10}$ Imidazol-1-yl, -NHPh, -OPh, $-N(CH_3)_2$ oder - $SCH_3$ und Ph Phenyl ist,
überführt, indem man eine Verbindung VII mit 1,1'-Thiocarbonyl-diimidazol, Phenylchlorothionoformiat, dimethylthiocarbamoylchlorid, Kohlendisulfid oder Phenylisothiocyanat in einem aprotischen Lösungsmittel, in Gegenwart einer Base umsetzt und die erhaltene Verbindung der Formel VIII in diejenige der Formel IX durch Umsetzung mit Tributylzinhydrid in einem aprotischen organischen Lösungsmittel, in Gegenwart eines Radikalinitiatoren überführt.

**9.** Pharmazeutisches Präparat, insbesondere für die Behandlung von hyperproliferativen Hauterkrankungen, wie Psoriasis, und für die Behandlung von Krankheiten der Talgdrüsen, wie Akne und seborrhoische Dermatitis, enthaltend eine wirksame Menge einer Verbindung der Formel I nach Anspruch 1 und einen pharmazeutisch akzeptablen Träger.

**10.** Verwendung einer Verbindung nach Anspruch 1, 2 oder 3 bei der Herstellung eines Medikamentes für die Behandlung von hyperproliferativen Hauterkrankungen, wie Psoriasis, und für die Behandlung von Krankheiten der Talgdrüsen wie Akne und seborrhoische Dermatitis.

## Revendications

**1.** Composé de formule

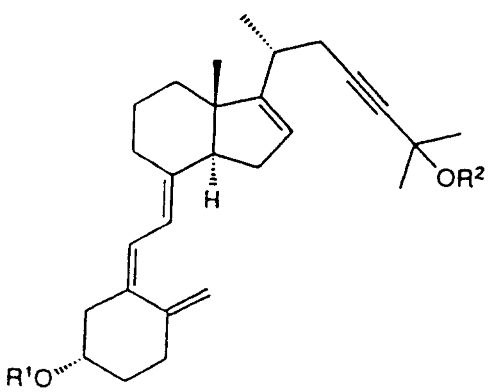

I

dans laquelle $R^1$ et $R^2$ sont un atome d'hydrogène ou un groupe acyle, étant entendu qu'un seul des radicaux $R^1$ et $R^2$ est un atome d'hydrogène.

**2.** Composé selon la revendication 1, dans lequel $R^1$ est $R^3CO$, $R^3$ est un groupe alkyle inférieur ou aryle, et $R^2$ est un atome d'hydrogène ou le groupe acétyle.

**3.** Composé selon la revendication 1, choisi parmi le diacétate de (3β,5Z,7E)-9,10-sécocholesta-5,7,10(19),16-tétraène-23-yne-3,25-diol et le 3-(4-phényl)benzoate de (3β,5Z,7E)-9,10-sécocholesta-5,7,10(19),16-tétraène-23-yne-3,25-diol.

**4.** Composé de formule

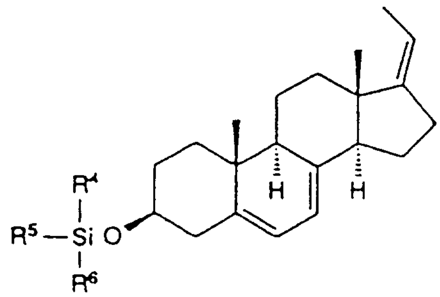

IV

dans laquelle $R^4$, $R^5$ et $R^6$ sont indépendamment un groupe alkyle en $C_1$-$C_6$ ou phényle, en particulier dans laquelle $R^4$ et $R^5$ représentent le groupe méthyle et $R^6$ est le groupe thexyle;

ou de formule

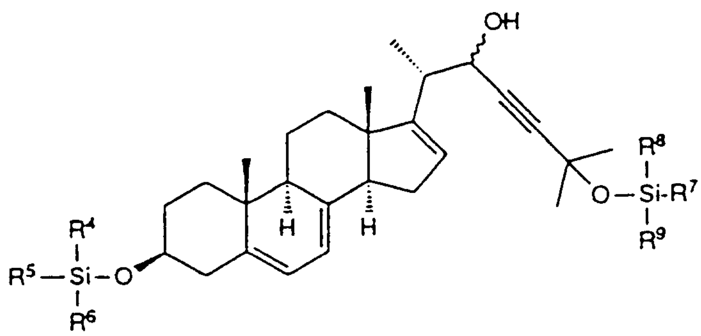

VII

dans laquelle $R^4$ à $R^9$ représentent indépendamment un groupe alkyle en $C_1$-$C_6$ ou phényle, en particulier dans laquelle $R^4$, $R^5$, $R^7$ et $R^8$ représentent le groupe méthyle, $R^6$ est le groupe thexyle, $R^9$ est le groupe tert-butyle.

ou de formule

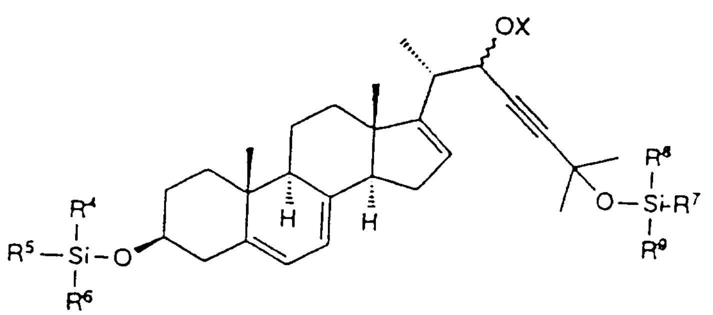

VIII

dans laquelle $R^4$ à $R^9$ représentent indépendamment un groupe alkyle en $C_1$-$C_6$ ou phényle, et X est un groupe -C(S)$R^{10}$ dans lequel $R^{10}$ est un groupe imidazol-1-yle, -NHPh, -OPh, -$N(CH_3)_2$ ou -$SCH_3$, en particulier dans laquelle $R^4$, $R^5$, $R^7$ et $R^8$ représentent le groupe méthyle, $R^6$ est le groupe thexyle, $R^9$ est le groupe tert-butyle, $R^{10}$ est un groupe -NHPh, et Ph est le groupe phényle, ou

de formule

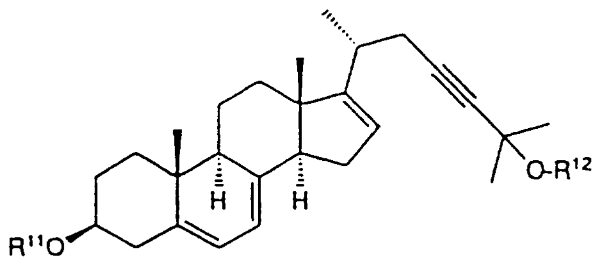

XIII

dans laquelle $R^{11}$ et $R^{12}$ représentent indépendamment un atome d'hydrogène ou un groupe -Si($R^4$,$R^5$,$R^6$) ou acyle, $R^4$, $R^5$ et $R^6$ représentent indépendamment un groupe alkyle en $C_1$-$C_6$ ou phényle, en particulier dans laquelle $R^{11}$ est le groupe diméthylthexylsilyle et $R^{12}$ est le groupe tert-butyldiméthylsilyle, ou dans laquelle $R^{11}$ et $R^{12}$ sont des atomes d'hydrogène, ou dans laquelle $R^{11}$ et $R^{12}$ sont des groupes acétyle; ou

de formule

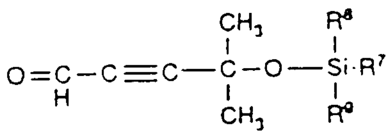

VI

dans laquelle $R^7$, $R^8$ et $R^9$ représentent indépendamment un groupe alkyle en $C_1$-$C_6$ ou phényle.

5. Composé selon la revendication 1, 2 ou 3, pour utilisation en tant qu'agent thérapeutiquement actif, en particulier pour le traitement de troubles hyperprolifératifs de la peau, tels que le psoriasis, et pour le traitement de maladies des glandes sébacées, telles que l'acné et la dermatite séborrhéique.

6. Procédé pour la préparation d'un composé de formule

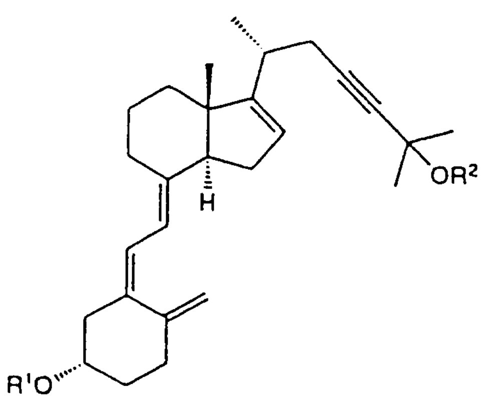

I

dans laquelle $R^1$ et $R^2$ représentent un atome d'hydrogène ou un groupe acyle,

comprenant la soumission d'un composé de formule

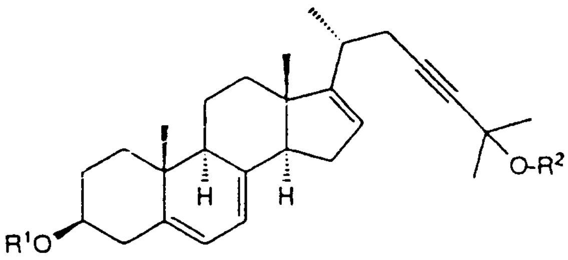

XI

dans laquelle $R^1$ et $R^2$ sont tels que décrits ci-dessus,

à une photolyse, de préférence par irradiation avec une lampe à vapeur de mercure à moyenne pression, en utilisant un 4-dialkylaminobenzoate, en particulier le 4-diméthylaminobenzoate d'éthyle, en tant que filtre pour arrêter la lumière d'environ 290-320 nm.

**7.** Procédé pour la préparation d'un composé de formule

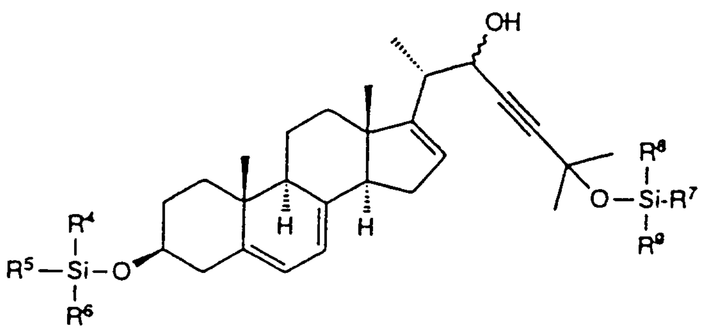

VII

dans laquelle $R^4$, $R^5$, $R^6$, $R^7$, $R^8$ et $R^9$ représentent indépendamment un groupe alkyle en $C_1$-$C_6$ ou phényle,

comprenant la mise en réaction d'un composé de formule

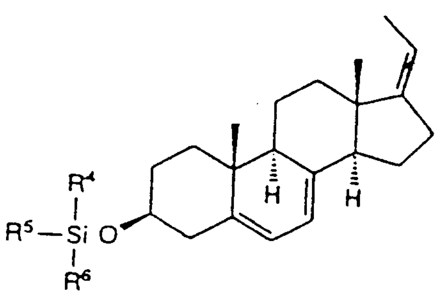

IV

dans laquelle $R^4$, $R^5$, $R^6$ sont tels que décrits ci-dessus

avec un composé de formule

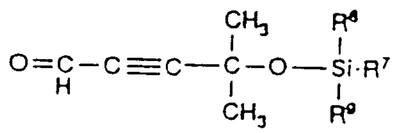

VI

dans laquelle $R^7$, $R^8$ et $R^9$ sont tels que décrits ci-dessus.

**8.** Procédé pour la préparation d'un composé de formule

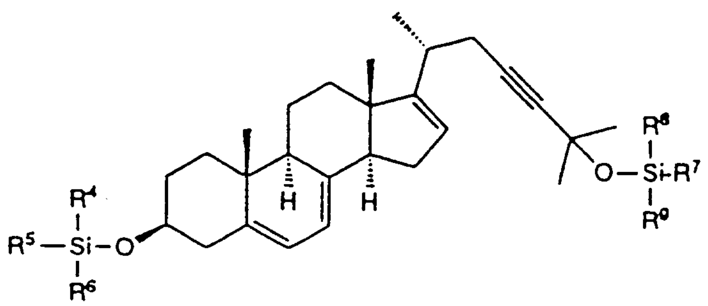

IX

dans laquelle $R^4$, $R^5$, $R^6$, $R^7$, $R^8$ et $R^9$ représentent indépendamment un groupe alkyle en $C_1$-$C_6$ ou phényle,

comprenant la conversion d'un composé de formule

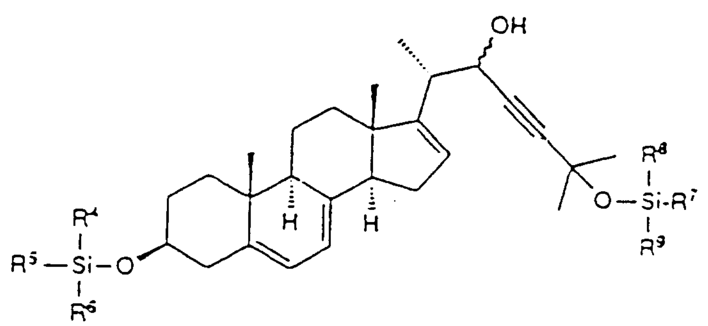

VII

dans laquelle $R^4$, $R^5$, $R^6$, $R^7$, $R^8$ et $R^9$ sont tels que décrits ci-dessus,

en un composé de formule

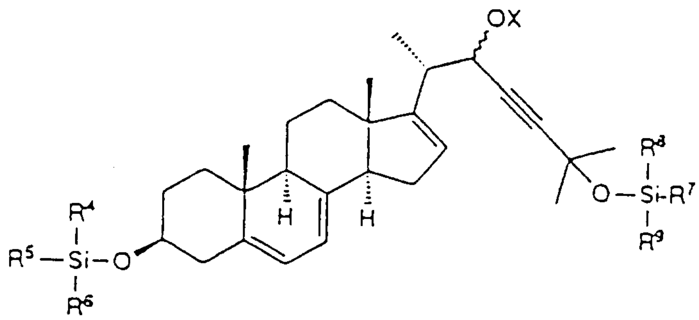

VIII

dans laquelle $R^4$, $R^5$, $R^6$, $R^7$, $R^8$ et $R^9$ sont tels que décrits ci-dessus, X est un groupe -C(S)$R^{10}$ dans lequel $R^{10}$ est le groupe imidazol-1-yle, -NHPh, -OPh, -$N(CH_3)_2$ ou -$SCH_3$, et Ph est le groupe phényle,

par mise en réaction d'un composé VII avec du 1,1'-thiocarbonyldiimidazole, du chlorothionoformiate de phényle, du chlorure de diméthylthiocarbamoyle, du disulfure de carbone ou du phénylisothiocyanate, dans un solvant aprotique, en présence d'une base, et conversion du composé de formule VIII obtenu en celui de formule IX, par une réaction avec de l'hydrure de tributylétain dans un solvant organique aprotique, en présence d'un amorceur de radicaux.

9. Composition pharmaceutique, en particulier pour le traitement de troubles hyperprolifératifs de la peau, tels que le psoriasis, et pour le traitement de maladies des glandes sébacées, telles que l'acné et la dermatite séborrhéique, comprenant une quantité efficace d'un composé de formule I selon la revendication 1, et un véhicule pharmaceutiquement acceptable.

10. Utilisation d'un composé tel que défini dans la revendication 1, 2 ou 3, pour la fabrication d'un médicament destiné au traitement de troubles hyperprolifératifs de la peau, tels que le psoriasis, et au traitement de maladies des glandes sébacées, telles que l'acné et la dermatite séborrhéique.